# EUROPÄISCHE PATENTSCHRIFT

(11) **EP 1 483 753 B1**
(45) Veröffentlichungstag und Bekanntmachung des Hinweises auf die Patenterteilung: **14.06.2006**
(21) Anmeldenummer: 03704427.8
(22) Anmeldetag: 18.01.2003
(51) Int. Cl.: G08B 21/16, G01N 33/00

(54) **METHANGAS, BEWACHUNGSANLAGE MIT ZUMINDEST EINER NGM (NATURAL GAS MONITOR), SENSOREINRICHTUNG**
METHANE GAS-MONITORING SYSTEM COMPRISING AT LEAST ONE NGM (NATURAL GAS MONITOR) SENSOR DEVICE
SYSTEME DE SURVEILLANCE DE GAZ METHANE COMPORTANT AU MOINS UN DISPOSITIF CAPTEUR NGM (SURVEILLANCE DU GAZ NATUREL)

(30) Priorität: 11.03.2002 DE 20203865 U
(43) Veröffentlichungstag der Anmeldung: 08.12.2004
(73) Patentinhaber: paragon AG, 33129 Delbrück (DE)
(72) Erfinder: FRERS, Klaus-Dieter, 33129 Delbrück (DE)
(74) Vertreter: Lelgemann, Karl-Heinz
(86) Internationale Anmeldenummer: PCT/EP2003/000466
(87) Internationale Veröffentlichungsnummer: WO 2003/077218

(56) Entgegenhaltungen:
- FR-A- 2 601 453

## Beschreibung

Die Erfindung bezieht sich auf eine Methangas-Überwachungsanlage nach dem Oberbegriff des Patentanspruchs 1.

Eine derartige Methangas-Überwachungsanlage ermöglicht eine sichere Detektion von Methangas bereits dann, wenn der Methangasgehalt des zu überwachenden Atmosphärevolumens deutlich unterhalb der Explosionsgrenze liegt. Da Methangas der Hauptbestandteil von in großem Ausmaß genutztem Erd- und Stadtgas ist, kommen derartige Methangas-Überwachungsanlagen häufig zur Unfallverhütung ud.dgl. zum Einsatz.

Die in derartigen Methangas-Überwachungsanlagen zum Einsatz kommenden Sensorelemente sind üblicherweise als Halbleitersensoren ausgebildet und haben damit grundsätzliche Schwächen hinsichtlich ihrer Langzeitstabilität, hinsichtlich ihres Empfindlichkeitsverlustes aufgrund chemischer Veränderungen des Sensormaterials und hinsichtlich der Langzeitdrift des Nullpunktes.

Die mit entsprechenden Sensorelementen ausgerüsteten NGM-Sensoreinheiten weisen daher möglicherweise nach einer gewissen Betriebszeit eine Unsicherheit auf. Ein Selbsttest der NGM-Sensoreinheit mit anschließender Warnung bei fehlerhaftem Sensorelement ist nur bedingt möglich. Langzeiteffekte, wie beispielsweise eine erhöhte Drift oder eine verringerte Empfindlichkeit des Sensorelements sind mit vertretbarem wirtschaftlichen Aufwand vor Ort nicht detektierbar.

Aus der FR-A-2 601 453 ist eine Methangas-Überwachungsanlage bekannt, bei der eine NGM-Sensoreinrichtung vorgesehen ist. Diese Sensoreinrichtung ist als solche als Modul ausgestaltet, wobei sie hinsichtlich ihrer Abmessungen die übliche Modulgröße von Bestandteilen elektrischer Anlagen oder aber die doppelte Modulgröße derselben aufweist. Mittels der NGM-Sensoreinrichtung ist ein Methangasgehalt erfaßbar und ein Alarmsignal optischer oder akustischer Art auslösbar, sofern ein für den Methangasgehalt vorgegebener Grenzwert durch das erfaßte Methangassignal überschritten wird.

Zu der NGM-Sensoreinrichtung gehört eine Sensoreinheit, die sich wiederum aus einem eigentlichen Sensorelement und einer das Meßsignal desselben verarbeitenden Auswerteschaltung zusammensetzt. Die Auswerteschaltung enthält sämtliche elektrischen und elektronischen Bestandteile, wie Komparatoren etc., die erforderlich sind, um ein Auslösesignal für einen optischen oder akustischen Signalgeber zu erzeugen. Des weiteren gehört eine Einrichtung zur Konstanthaltung der Energieversorgung zu der bekannten NGM-Sensoreinheit, die darüber hinaus auch einen Transformator beinhalten kann.

Die gesamte Sensoreinrichtung unter Einschluß der NGM-Sensoreinheit ist innerhalb eines Gehäuses angeordnet, das an der Vorderseite mittels eines Gitterelements abgeschlossen ist.

Die Entfernung des eigentlichen Sensorelements aus der Sensoreinrichtung ist ohne weiteres nicht möglich, da hierzu zunächst die die Vorderseite des Gehäuses abschließende Gitterwandung entfernt werden muß, wobei darüber hinaus das eigentliche Sensorelement bei der bekannten Sensoreinrichtung fest mit anderen Bestandteilen derselben, die nicht unmittelbar mit dem Sensorelement zusammenwirken, verbunden ist.

Auch bei der aus der FR-A-2601453 bekannten Methangas-Überwachungsanlage kommen üblicherweise Sensorelemente zum Einsatz, die als Halbleitersensoren ausgebildet sind und damit die eingangs bereits geschilderten Nachteile aufweisen.

Der Erfindung liegt die Aufgabe zugrunde, die gattungsgemäße Methangas-Überwachungsanlage derart weiterzubilden, daß mit einem minimalen Aufwand ein langfristig wirksamer Schutz gegen unbemerkte Sensorausfälle sicher möglich ist.

Diese Aufgabe wird erfindungsgemäß durch die im kennzeichnenden Teil des Patentanspruchs 1 angegebenen Merkmale gelöst. Die Betriebssicherheit und die Meßgenauigkeit einer derart als Modulteil ausgebildeten NGM-Sensoreinheit läßt sich über einen vorgebbaren Zeitraum, beispielsweise für ein Jahr, garantieren. Aufgrund der Ausgestaltung als Modulteil kann dann in den entsprechend vorgegebenen Zeitabständen planmäßig ein Austausch der NGM-Sensoreinheiten in der Methangas-Überwachungsanlage vorgenommen werden, wodurch eine größtmögliche Sicherheit der Methangas-Überwachungsanlage gewährleistet ist.

Die unmittelbar mit dem eigentlichen Sensorelement zusammenwirkenden Bestandteile der NGM-Sensoreinrichtung sind zu der modulartig gestalteten NGM-Sensoreinheit zusammengefaßt. Die innerhalb des Gehäuses der NGM-Sensoreinheit aufgenommene Auswerteschaltung enthält ausschließlich diejenigen Funktionen, die unmittelbar der Bearbeitung des vom eigentlichen Sensorelement erfaßten Meßsignals dienen. Irgendwelche weiteren elektrischen oder elektronischen Bauteile, die beispielsweise dem Entscheidungsprozeß bezüglich der Herbeiführung oder der Nichtherbeiführung eines optischen oder akustischen Warnsignals dienen, sind nicht Bestandteil der innerhalb des Gehäuses der NGM-Sensoreinheit aufgenommenen Auswerteschaltung. Bei einem Austausch der NGM-Sensoreinheit verbleiben somit die dieser nachgeschalteten elektrischen und elektronischen Bestandteile der NGM-Sensoreinrichtung in letzterer.

Die aus der Methangas-Überwachungsanlage entnommene, als Modulteil ausgebildete NGM-Sensoreinheit wird zentral, beispielsweise werksseitig, überprüft, kalibriert und ggf. neu justiert, wonach diese NGM-Sensoreinheit ohne Sicherheitsverlust erneut für den vorgebbaren Zeitraum in die Methangas-Überwachungsanlage eingebaut werden kann. Für diese NGM-Sensoreinheiten wird erfindungsgemäß somit ein Wiederverwertungskreislauf geschaffen, mittels dem erhebliche Einsparungspotentiale realisiert werden können.

Im Falle der erfindungsgemäßen Methangas-Überwachungsanlage können unbemerkte Funktionsverluste der NGM-Sensoreinheiten zuverlässig vermieden werden.

Das Sensorelement ist vorteilhaft als Standardhalbleiterelement für die Detektion verschiedener gasförmiger Kohlenwasserstoffe ausgelegt, wobei die Auswerteschaltung zweckmäßigerweise so ausgebildet ist, daß die NGM-Sensoreinheit als solche ausschließlich ober nahezu ausschließlich den Methangasgehalt erfaßt.

Jede NGM-Sensoreinheit kann gemäß einer vorteilhaften Ausführungsform der erfindungsgemäßen Methangas-Überwachungsanlage eine Sensorverriegelung und einen Verdrehschutz aufweisen.

Wenn jede NGM-Sensoreinheit nach dem Einsetzen in die NGM-Sensoreinrichtung verriegelt ist, kann die als Modulteil ausgebildete NGM-Sensoreinheit nach der Verriegelung mit einer Prüfplakette plombiert werden, auf der das nächste Austauschdatum angezeigt werden kann.

Im folgenden wird die Erfindung anhand einer Ausführungsform unter Bezugnahme auf die Zeichnung näher erläutert.
Es zeigen:
- Figur 1: eine Vorderansicht einer als Modulteil ausgebildeten NGM-Sensoreinheit;
- Figur 2: den Schnitt B - B in Figur 1; und
- Figur 3: eine Draufsicht auf die in Figur 1 gezeigte Ausführungsform der NGM-Sensoreinheit.

Eine erfindungsgemäße Methangas-Überwachungsanlage hat zumindest eine, vorzugsweise mehrere, NGM-(Natural Gas Monitor)-Sensoreinrichtungen, die bestimmte Luftvolumina daraufhin überwachen, ob in ihnen ein maximal zulässiger Methangasgehalt überschritten wird. Mittels dieser im weiteren nicht dargestellten NGM-Sensoreinrichtungen ist es möglich, ein Alarmsignal, bei dem es sich um ein optisches, ein akustisches oder ein anderes Alarmsignal handeln kann, auszulösen, wenn der Methangasgehalt in dem zu überwachenden Gasvolumen den maximal zulässigen Grenzwert überschreitet.

Jede der im übrigen nicht dargestellten NGM-Sensoreinrichtungen weist eine NGM-Sensoreinheit 1 auf, mittels der die eigentliche Gasdetektion innerhalb des zu überwachenden Volumens bewerkstelligt wird.

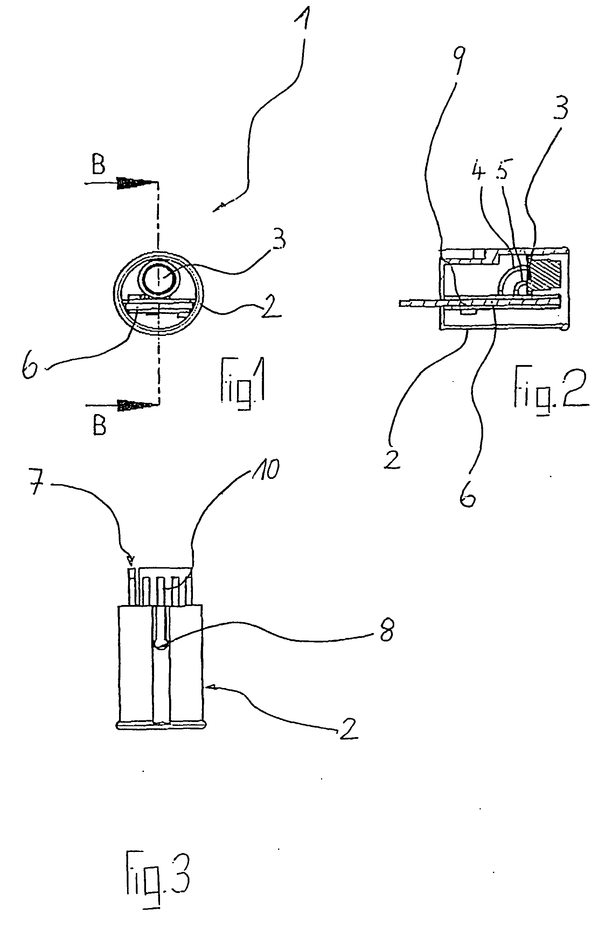

Die anhand der Figuren 1 bis 3 näher gezeigte NGM-Sensoreinheit 1 hat ein Kunststoffgehäuse 2, in dem das eigentliche Sensorelement 3 angeordnet ist. Das Sensorelement 3 ist als Standardhalbleitersensor ausgebildet und entsprechend für die Detektion beliebiger gasförmiger Kohlenwasserstoffe geeignet. Eine selektive Erfassung des Methangehalts innerhalb des zu überwachenden Volumens ist ohne weitere Maßnahmen mit dem als Standardhalbleitersensor ausgebildeten Sensorelement 3 nicht möglich.

Das Sensorelement 3 ist mittels Anschlüssen 4, 5 an eine auf einer Platine 6 realisierten Auswerteschaltung angeschlossen. Die Platine 6 bzw. die auf ihr realisierte Auswerteschaltung 6 ist so ausgelegt, daß mittels ihrer intelligenten Auswerteelektronik die Empfindlichkeit für alle Kohlenwasserstoffe außer Methan minimiert ist. Die Empfindlichkeit des Standardhalbleitersensors bzw. des Sensorelements 3 in bezug auf Methangas wird aufgrund der Ausgestaltung der mittels der Platine 6 realisierten Auswerteschaltung beibehalten.

Mittels einer ausgangsseitig der Auswerteschaltung 6 vorgesehenen Schnittstelle wird das seitens des Sensorelements 3 erfaßte bzw. erzeugte Meßsignal in mittels der Auswerteschaltung 6 ausgewerteter Form an die NGM-Sensoreinrichtung, die in den Figuren 1 bis 3 nicht dargestellt ist, weitergeleitet.

Das Kunststoffgehäuse 2 der NGM-Sensoreinheit 1 ist mit einer Sensorverriegelung und einem Verdrehschutz 8 versehen. Innerhalb des Kunststoffgehäuses 2 ist für die Platine 6 bzw. die mittels ihr realisierte Auswerteschaltung ein Bestückungsbauraum 9 ausgebildet, in dem die Platine bzw. Auswerteschaltung 6 teilweise aufgenommen ist und aus dem sie mit ihren Kontaktflächen 10 vorsteht.

Die vorstehend geschilderte NGM-Sensoreinheit 1 ist als Modulteil ausgebildet, welches in die in den Figuren nicht dargestellte NGM-Sensoreinrichtung in einfacher Weise einsetzbar und aus dieser herausnehmbar ist. Die als Modulteil ausgebildete NGM-Sensoreinheit 1 wird entsprechend mit einem geringen Aufwand in regelmäßigen Zeitabständen, beispielsweise jährlich, ausgetauscht, wobei sie durch eine kalibrierte NGM-Sensoreinheit 1 ersetzt wird. Die der NGM-Sensoreinrichtung entnommene NGM-Sensoreinheit 1 wird werksseitig überprüft, kalibriert und ggf. neu justiert, wonach sie erneut ohne Sicherheitsverlust für einen weiteren Betriebszeitraum in die NGM-Sensoreinrichtung einbaubar ist.

Nach ihrem Einbau in die NGM-Sensoreinrichtung wird die als Modulteil ausgebildete NGM-Sensoreinheit 1 mittels einer Prüfplakette plombiert, wobei auf der Prüfplakette das nächste Austausch- bzw. Kalibrierdatum angezeigt wird.

## Patentansprüche

1. Methangas-Überwachungsanlage, mit zumindest einer NGM (Natural Gas Monitor)-Sensoreinrichtung, mittels der ein Methangasgehalt erfaßbar und, sofern der erfaßte Methangasgehalt einen maximal zulässigen Grenzwert überschreitet, ein Alarmsignal auslösbar ist und die zur Gasdetektion eine NGM-Sensoreinheit (1) mit einem Sensorelement (3) und einer Auswerteschaltung (6) aufweist, **dadurch gekennzeichnet, daß** die NGM-Sensoreinheit (1) der NGM-Sensoreinrichtung ein Kunststoffgehäuse (2), in dem das Sensorelement (3) und die Auswerteschaltung (6) angeordnet sind, und eine Schnittstelle (7) aufweist, mittels der das seitens des Sensorelements (3) erfaßte bzw. erzeugte Meßsignal in mittels der Auswerteschaltung (6) ausgewerteter Form an die NGM-Sensoreinrichtung weiterleitbar ist, und daß die das Sensorelement (3), die Auswerteschaltung (6), die Schnittstelle (7) und das Kunststoffgehäuse (2) aufweisende NGM-Sensoreinheit (1) als in die NGM-Sensoreinrichtung einsetzbares und aus dieser herausnehmbares Modulteil ausgebildet ist.

2. Methangas-Überwachungsanlage nach Anspruch 1, bei der das Sensorelement (3) als Standardhalbleitersensorelement für die Detektion verschiedener gasförmiger Kohlenwasserstoffe und die Auswerteschaltung (6) so ausgebildet ist, daß die NGM-Sensoreinheit (1) ausschließlich oder nahezu ausschließlich den Methangasgehalt erfaßt.

3. Methangas-Überwachungsanlage nach Anspruch 1 oder 2, bei der jede NGM-Sensoreinheit (1) eine Sensorverriegelung und einen Verdrehschutz (8) aufweist.

4. Methangas-Überwachungsanlage nach einem der Ansprüche 1 bis 3, bei der jede NGM-Sensoreinheit (1) nach dem Einsetzen in die NGM-Sensoreinrichtung plombierbar ist.

5. Methangas-Überwachungsanlage nach einem der Ansprüche 1 bis 4, deren NGM-Sensoreinheit (1) prüf-, kalibrier- und wiederverwertbar ausgebildet ist.

## Claims

1. A methane gas monitoring system comprising at least one NGM (natural gas monitor) sensor device, which can detect a methane gas content and if the detected methane gas content exceeds a maximum permissible limiting value, triggers an alarm signal and which has an NGM sensor unit (1) for gas detection comprising a sensor element (3) and an evaluating circuit (6), **characterised in that** the NGM sensor unit (1) of the NGM sensor device has a plastic housing (2) in which the sensor element (3) and the evaluation circuit (6) are arranged, and an interface (7) by which means the measured signal detected or produced by the sensor element (3) is passed in a form which can be evaluated by means of the evaluation circuit (6) to the NGM sensor device and that the NGM sensor unit (1) comprising the sensor element (3), the evaluation circuit (6), the interface (7) and the plastic housing (2) is constructed as a module part which can be inserted into the NGM sensor device and withdrawn therefrom.

2. The methane gas monitoring system according to claim 1, wherein the sensor element (3) is constructed as a standard semiconductor element for the detection of various gaseous hydrocarbons and the evaluation circuit (6) is constructed so that the NGM sensor unit (1) exclusively or almost exclusively detects the methane gas content.

3. The methane gas monitoring system according to claim 1 or 2, wherein each NGM sensor unit (1) comprises a sensor lock and an anti-rotation device (8).

4. The methane gas monitoring system according to any one of claims 1 to 3, wherein each NGM sensor unit (1) is lead-sealed after insertion into the NGM sensor device.

5. The methane gas monitoring system according to any one of claims 1 to 4, whose sensor unit (1) is constructed so that it can be tested, calibrated and reused.

## Revendications

1. Installation de surveillance du méthane, avec au moins un dispositif de détection NGM (Natural Gas Monitor - moniteur à gaz naturel), au moyen duquel une teneur en méthane peut être détectée, dès que la teneur en méthane détectée dépasse une valeur limite maximale autorisée, un signal d'alarme peut être déclenché et qui pour la détection de gaz, comporte une unité de détection NGM (1) avec un élément détecteur (3) et un circuit d'évaluation (6), **caractérisée en ce que** l'unité de détection NGM (1) de l'installation de détection NGM comporte un boîtier en matière plastique (2) dans lequel l'élément détecteur (3) et le circuit d'évaluation (6) sont disposés et une interface (7), au moyen de laquelle le signal de mesure détecté ou généré du côté de l'élément détecteur (3) peut être retransmis à l'installation de détection NGM, sous une forme évaluée au moyen du circuit d'évaluation (6) et **en ce que** l'unité de détection NGM comportant l'élément détecteur (3), le circuit d'évaluation (6), l'interface (7) et le boîtier en matière plastique (2) est conçue en tant qu'élément modulaire pouvant être intégré dans le dispositif de détection NGM et retiré de ce dernier.

2. Installation de surveillance du méthane selon la revendication 1, dans laquelle l'élément détecteur (3) est conçu en tant qu'élément détecteur à semi conducteur standard pour la détection de différents hydrocarbures gazeux et le circuit d'évaluation (6) est conçu de façon telle, que l'unité de détection NGM (1) détecte exclusivement ou presque exclusivement la teneur en méthane.

3. Installation de surveillance du méthane selon la revendication 1 ou 2, dans laquelle chaque unité de détection NGM (1) comporte un verrouillage de détecteur et une protection antitorsion (8).

4. Installation de surveillance du méthane selon l'une quelconque des revendications 1 à 3, dans laquelle chaque unité de détection NGM (1) peut être plombée, après avoir été insérée dans le dispositif de détection NGM.

5. Installation de surveillance du méthane selon l'une quelconque des revendications 1 à 4, dont l'unité de détection NGM (1) est conçue de façon à pouvoir être contrôlée, calibrée et recyclée.
